# EUROPEAN PATENT APPLICATION

(11) **EP 0 895 774 A2**
(43) Date of publication of application: **10.02.1999**
(21) Application number: 98114735.8
(22) Date of filing: 05.08.1998
(51) Int. Cl.: A61K 6/083

(54) **Radiopaque dental composites**

(30) Priority: 06.08.1997 US 907177
(71) Applicant: JENERIC/PENTRON Incorporated, Wallingford, Connecticut 06492 (US)
(72) Inventor: Schulman, Martin, Orange CT 06477 (US); Prasad, Arun, Cheshire CT 06410 (US)
(74) Representative: Maureau, Philippe

(57) **Abstract**

A fiber-reinforced composite material fro dental restorations, wherein the reinforcing glass fibers comprise a radiopacity-imparting agent, and are therefore inherently radiopaque. Preferably, the glass fibers comprise tantalum oxide, in an amount from about 5 to 60 percent by weight of the total glass composition and most preferably from about 10 to about 25 percent by weight of the total glass composition. The radiopaque fiber-reinforced composite is particularly useful as a structural component for bridges or a tooth restoration after endodontic treatment.

## Description

### Background of the Invention

### 1. Field of the Invention:

This invention relates to dental composite materials. In particular, this invention relates to fibers for reinforcement of dental composite materials, wherein the fibers are radiopaque.

### 2. Brief Discussion of the Prior Art

Fiber-reinforced composites have found increasing use in the field of materials for dental restorations, and are described, for example, in U.S. Patent Nos. 4,717,341 and 4,894,012 to Goldberg et al., as well as U.S. Patent No. 4,107,845 to Lee, Jr. et al. Fiber-reinforced composites generally comprise at least two components, a polymeric matrix and fibers embedded within the matrix. The polymeric matrix may be selected from those known for use in composite dental materials, for example polyamides, polyesters, polyolefins, polyimides, polyarylates, polyurethanes, vinyl esters or epoxy-based materials. The fibers used to reinforce composite material may comprise glass, carbon, or polymer fibers such as polyaramide and polyethylene, as well as other natural and synthetic fibers.

Fiber reinforced composite materials provides several advantages, most notably increased strength and stiffness. As described in U.S. Patent Nos. 4,717,341 and 4,894,012 to Goldberg et al., such materials accordingly are used as structural components in a variety of dental appliances, taking the form of bars, wires, beams, posts, clasps, and laminates for use in traditional bridges, crowns, artificial teeth, dentures, implants, veneers, and the like. They have also been used in connection orthodontic retainers, bridges, space maintainers, splints, and the like. In these applications, the fibers preferably take the form of long, continuous filaments, although the filaments may be as short as 3 to 4 millimeters. Shorter fibers of uniform or random length might also be employed. Where the composites take the form of elongated wires, the fibers are at least partially aligned and oriented along the longitudinal dimensions of the wire. However, depending on the end use of the composite material, the fibers may also be otherwise oriented, including being normal or perpendicular to that dimension.

One drawback to use of fiber-reinforced composites, however, has been a very low level, or lack of inherent radiopacity, that is, contrast under X-ray. X-ray contrast provides a non-destructive method of detection of recurrent caries, growth of neoplasms, and other tissue disorders. Preferably, dental restorations and surgical implants should possess sufficient X-ray contrast such that the position and boundary area of the implanted material be clearly delineated.

A number of methods for providing X-ray opacity to dental composite materials have been described. Metallic dental restorations are inherently radiopaque. X-ray opaque composites have been prepared by the incorporation of finely divided X-ray opaque fillers into the polymeric matrix, for example barium-containing glasses (see, e.g., U.S. Patent No. 3,539,526); zinc-containing glasses (see, e.g., U.S. Patent Re. 32,299); glasses containing rare earth elements such as strontium, tantalum, and lanthanum (see, e.g., U.S. Patent No. 4,017,454); zeolitic alumino silicates supporting various radiopaque metals (see U.S Patent No. 4,375,967); or polymer beads containing alkyl iodides (see U.S. Patent No. 4,302,376). However, effective use of radiopaque fillers in the polymeric matrix requires proper selection of type of filler, as well as the amount. The type and amount of filler required to impart radiopacity to a fiber-reinforced composite may well be incompatible with the requirements of the type and amount of filler (if any) required for effective use of fiber-reinforced composite materials. Accordingly, there remains a need for fiber reinforcement of dental materials wherein the fibers possess inherent radiopacity.

### Summary of the Invention:

The above-described drawbacks and deficiencies of the prior art are alleviated by the fiber-reinforced composite materials in accordance with the present invention, wherein reinforcing glass fibers are comprise an effective amount of a radiopacity-imparting agent, and are therefore inherently radiopaque. The preferred radiopacity-imparting agent is tantalum oxide.

In an alternative embodiment, the present invention comprises a fiber-reinforced composite in the form of a bar for reinforcement of bridges, wherein the fiber reinforcement comprises glass and an effective amount of a radiopacity-imparting agent. In still another embodiment, the present invention comprises a fiber-reinforced composite in the form of a post for reinforcement of a tooth restoration after endodontic treatment, wherein the fiber reinforcement comprises glass and an effective amount of a radiopacity-imparting agent.

### Brief Description of the Drawings:

FIGURE 1 is a perspective view, partially in section, of a bridge using a bar comprising the radiopaque fiber-reinforced composite in accordance with the present invention.
FIGURE 2 is a sectional view of a restored endodontically-treated tooth using a post comprising the radiopaque fiber-reinforced composite in accordance with the present invention.

### Detailed Description of the Invention:

The fiber-reinforced composite material in accordance with the present invention comprises a polymeric matrix and inherently radiopaque glass reinforcing fibers within the matrix. The fiber-reinforced composites are advantageously used as a structural component for dental restorations such as bridges and endodontically-treated tooth restorations.

The polymeric matrix element of the composite is selected from those known in the art of dental materials, including but not being limited to polyamides, polyesters, polyolefins, polyimides, polyarylates, polyurethanes, vinyl esters or epoxy-based materials. Other polymeric matrices include styrenes, styrene acrylonitriles, ABS polymers, polysulfones, polyacetals, polycarbonates, polyphenylene sulfides, and the like.

Preferred materials include those based on acrylic and methacrylic monomers, for, example those disclosed in U.S. Pat. Nos. 3,066,112, 3,179,623, and 3,194,784 to Bowen; U.S. Patent Nos. 3,751,399 and 3,926,906 to Lee et al.; and commonly assigned U.S. Pat. Nos. 5,276,068 to Waknine, all of which are herein incorporated by reference in their entirety. An especially preferred methacrylate monomer is the condensation product of bisphenol A and glycidyl methacrylate, 2,2'-bis [4-(3-methacryloxy-2-hydroxy propoxy)-phenyl]-propane (hereinafter abbreviated "BIS-GMA"). Polyurethane dimethacrylates (hereinafter abbreviated to PUDMA) are also commonly-used principal polymers suitable for use in the present invention.

The polymer matrix typically includes polymerization initiators, polymerization accelerators, ultra-violet light absorbers, anti-oxidants, and other additives well known in the art. The polymer matrices may be visible light curable, self-curing, dual curing, and vacuum, heat, and pressure curable compositions as well as any combination thereof. The visible light curable compositions include the usual polymerization initiators, polymerization accelerators, ultraviolet absorbers, fluorescent whitening agents, and the like. In the self-curing compositions, the polymerization accelerator can be included in the resinous composition which is used for pretreating the exposed dentin. The heat and pressure curable compositions, which are generally filled compositions, include, in addition to the monomeric components, a heat cure initiator such as benzoyl peroxide, 1,1'-azobis(cyclohexanecarbo-nitrile), or other free radical initiators.

Depending on the use, the polymer matrix may further comprise at least one filler known in the art and used in dental restorative materials, the amount of such filler being determined by the specific use of the fiber-reinforced composite. For example, where the fiber-reinforced composite is used as a structural component, no or relatively little additional filler may be present in the polymeric matrix, i.e., up to ten percent by weight of the polymeric matrix.

Suitable fillers are those capable of being covalently bonded to the polymeric matrix itself or to a coupling agent which is covalently bonded to both. Examples of suitable filling materials include but are not limited to those known in the art such as silica, silicate glass, quartz, barium silicate, strontium silicate, barium borosilicate, strontium borosilicate, borosilicate, lithium silicate, amorphous silica, ammoniated or deammoniated calcium phosphate and alumina, zirconia, tin oxide, and titania. Particularly suitable fillers for dental filling-type materials prepared in accordance with this invention are those having a particle size ranging from about 0.1-5.0 µm with a silicate colloid of 0.001 to about 0.07 microns and prepared by a series of milling steps comprising wet milling in an aqueous medium, surface etch milling and silanizing milling in a silane solution. Some of the aforementioned inorganic filling materials are disclosed in commonly-assigned U.S. Pat. Nos. 4,544,359 and No. 4,547,531 to Waknine, the pertinent portions of which are incorporated herein by reference.

The reinforcing fiber element of the composite comprises glass and an effective amount of a radiopacity-imparting agent. Suitable glasses include those known in the art, including but not being limited to the compositions set forth in the Table below. A preferred glass formulation is known in the art as "S-2 Glass". In general, the radiopacity-imparting agent will be incorporated into the glass replacing one or a combination of Al₂O₃ or B₂O₃.

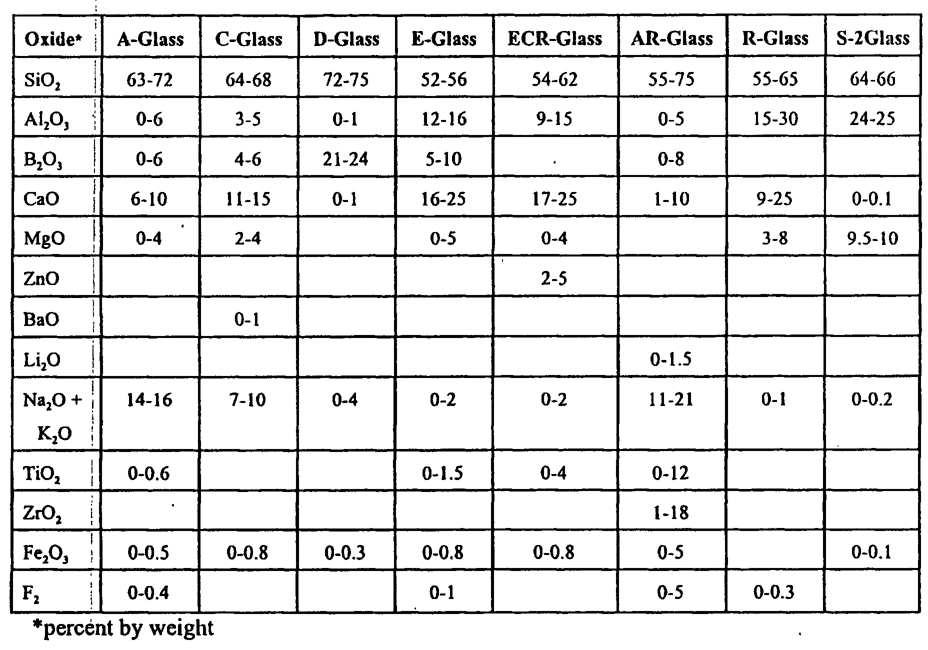

Radiopacity-imparting agents may include those known in the art, for example, the elements calcium, barium, bismuth, magnesium, and rare earth metals having an atomic number of 58-71, or oxides, carbonates, fluorides, or nitrates thereof. Preferred radiopacity-imparting agents comprise the oxides, carbonates, nitrates, or fluorides of lanthanum, hafnium, strontium, tantalum, ytterbium, yttrium, zirconium, or zinc, including but not being limited to La₂O₃, La₂(CO₃)₃, SrO, SrCO₃, SrF₂, Ta₂O₅, TaF₅, Y₂O₃,YF₃, ZnO, and ZrO, ZrCO₃. Particularly preferred radiopacity-imparting agents comprise the oxides, carbonates, or fluorides of lanthanum, strontium, or tantalum. Combinations of the foregoing may also be used.

The radiopacity-imparting agent is incorporated into the glass fibers by means known in the art. The amount of radiopacity-imparting agent must be effective to provide radiopacity to the fiber-reinforced composite. Generally, effective radiopacity will approximate the radiopacity of natural dentin or enamel, and retain such radiopacity throughout the life of the dental restoration. The amount of radiopacity-imparting agent will therefore be determined empirically, depending on factors such as the inherent radiopacity of the radiopacity-imparting agent, the amount of reinforcing glass fiber used, and the identity of the other components of the glass (for example CaO, MgO, and ZnO, which may themselves provide some degree of radiopacity). Generally, the radiopacity-imparting agent will comprise from about 5 to about 60 percent by weight of the total glass composition, and preferably from about 10 to about 25, percent by weight of the total glass composition.

The inherently radiopaque composites in accordance with the present invention are used as structural components in a variety of dental appliances, taking the form of bars, wires, beams, posts, clasps, and laminates for use in traditional bridges, crowns, artificial teeth, dentures, implants, veneers, and the like, or in connection orthodontic retainers, bridges, space maintainers, splints, and the like. Where the composite is used as a structural component, the inherently radiopaque glass fibers preferably take the form of long, continuous filaments, although the filaments may be as short as 3 to 4 millimeters. Shorter fibers of uniform or random length might also be employed. Where the composites take the form of elongated wires, the fibers are at least partially aligned and oriented along the longitudinal dimensions of the wire. However, depending on the end use of the composite material, the fibers may also be otherwise oriented, including being normal or perpendicular to that dimension. The amount of reinforcing fibers use will depend on the particular application, but preferably comprises at least about 20% by weight of the composite material. Preferably, the reinforcing fibers are used in accordance with U.S. Patent Nos. 4,717,341 and 4,894,012 to Goldberg et al., the relevant portions of which are herein incorporated by reference.

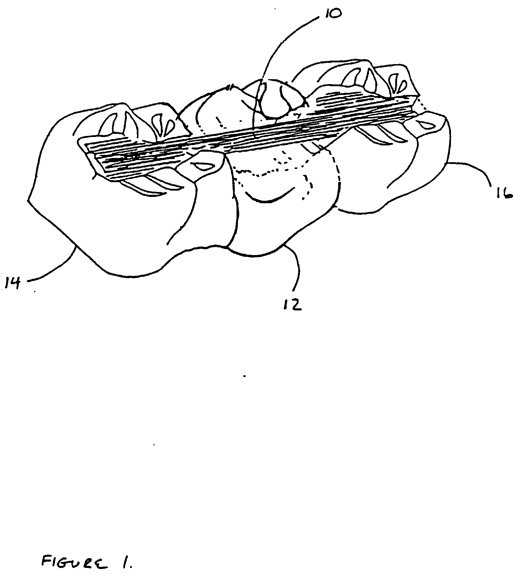

Referring now to FIGURE 1, a preferred embodiment of the structural component of the present invention is shown, wherein the radiopaque glass-reinforced composite is in the form a bar 10 providing structural support for a bridge 12 between teeth 14, 16. The cross-section of bar 10 may be rectangular, rhomboidal, ovoidal, cylindrical, or of any other cross-sectional configuration effective to provide strength and stiffness to the finished bridge. Bar 10 may be provided to the dentist or technician as a prefabricated composite, or it may be fabricated by the dentist or technician from the inherently radiopaque glass fibers described above and an appropriate polymeric matrix.

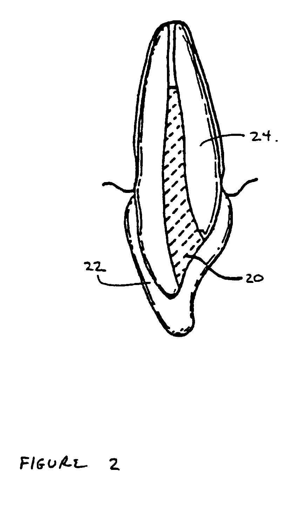

FIGURE 2 shows an alternative preferred embodiment of the structural component according to the present invention, wherein the radiopaque glass-reinforced composite is in the form a post 20 providing structural support for a crown 22 fitted to an endodontically-treated tooth 24. The cross-sectional shape of the post may also be rectangular, rhomboidal, ovoidal, cylindrical, or any other shape effective to provide strength to the tooth restoration. Suitable configurations for posts are well-known in the art.

Post 20 may also be provided to the dentist or technician as a prefabricated composite, or fabricated by the dentist or technician from the inherently radiopaque glass fibers described above and an appropriate polymeric matrix. In a particularly preferred embodiment, the composite post is supplied or formed by the technician and only partially cured, such that the polymeric matrix retains a degree of malleability. The composite post is then fitted to the endodontically treated tooth so as to conform the post to the tooth cavity, and fully cured. This process allows ready "custom-fitting" of a post to any endodontically-treated tooth.

While preferred embodiments have been shown and described, various modifications and substitutions may be made thereto without departing from the spirit and scope of the invention. Accordingly, it is to be understood that the present invention has been described by way of illustrations and not limitation.

## Claims

1. A fiber-reinforced dental composite comprising
a polymeric matrix; and
inherently radiopaque glass fibers in an amount effective to provide X-ray contrast to the dental composite.

2. The fiber-reinforced dental composite of claim 1, wherein
the radiopacity-imparting agent is at least one of the element or an oxide, carbonate, nitrate, or fluoride of calcium, barium, bismuth, magnesium, rare earth metals having an atomic number of 58-71, lanthanum, hafnium, strontium, tantalum, ytterbium, yttrium, zirconium, or zinc, or a mixture thereof.

3. The fiber-reinforced dental composite of claim 1 or 2, wherein
the radiopacity-imparting agent is at least one of an oxide, carbonate, nitrate, or fluoride of lanthanum, strontium, tantalum, ytterbium, yttrium, zinc, zirconium, or a mixture thereof.

4. The fiber-reinforced dental composite of claim 1,2, or 3, wherein
the radiopacity-imparting agent is tantalum oxide.

5. The fiber-reinforced dental composite of claim 1,2,3, or 4, wherein
the radiopacity-imparting agent is present in an amount in the range from about 5 to about 60 percent by weight of the total glass composition.

6. The fiber-reinforced dental composite of claim 1, 2, 3, 4, or 5, wherein
the glass comprises silicon dioxide in an amount of about 64-66 percent by weight, aluminum oxide in an amount of about 24-25 percent by weight, calcium oxide in an amount of about 0-0.1 percent by weight, magnesium oxide in an amount of about 9.5-10 percent by weight, sodium oxide and potassium oxide in combination in an amount of about 0-0.2 percent by weight, and iron oxide in an amount of about 0-0.1 percent by weight.

7. The fiber-reinforced dental composite of claim 1, 2, 3, 4, 5, or 6, wherein
the composite is in the form of a structural component for a dental restoration, the structural component having a length greater than its average diameter.

8. The fiber-reinforced dental composite of claim 1, 2, 3, 4, 5, 6, or 7, wherein
the structural component is in the shape of a bar for effective strengthening of a bridge or in the shape of a post for effective strengthening of an endodontically-treated tooth.

9. The fiber-reinforced dental material of claim 1, 2, 3, 4, 5, 6, 7, or 8, wherein
the polymeric matrix of the composite is partially cured.

10. A dental restoration comprising the fiber-reinforced ddental composte of claim 1, 2, 3, 4, 5, 6, 7, 8, or 9.

11. A method of forming a structural component of a restoration for an endodontically-treated tooth, comprising
forming a composite post (20) comprising monomers forming a polymeric matrix upon cure and inherently radiopaque fibers in an amount effective to provide X-ray contrast to the dental composite, wherein the radiopaque fibers comprise a glass and a radiopacity-imparting agent;
partially curing the monomers;
placing the post in the endodontically-treated tooth (24), thereby conforming the post to the tooth; and
fully curing the post, thereby forming a custom-fit post for reinforcement of the restoration.

12. The method of claim 11, wherein
the radiopacity-imparting agent is at least one of an element or oxide, carbonate, nitrate, or fluoride of calcium, barium, bismuth, magnesium, rare earth metals having an atomic number of 58-71, lanthanum, hafnium, strontium, tantalum, ytterbium, yttrium, zirconium, or zinc, or a mixture thereof.

13. The method of claim 11 or 12, wherein
the radiopacity-imparting agent is at least one of an oxide, carbonate, nitrate, or fluoride of lanthanum, strontium, tantalum, ytterbium, yttrium, zinc, or zirconium, or a mixture thereof.

14. The method of claim 11, 12, or 13, wherein
the radiopacity-imparting agent is present in an amount in the range from about 5 to about 60 percent by weight of the total glass composition.

15. The method of claim 11, 12, 13, or 14, wherein
the glass comprises silicon dioxide in an amount of about 64-66 percent by weight, aluminum oxide in an amount of about 24-25 percent by weight, calcium oxide in an amount of about 0-1.1 percent by weight, magnesium oxide in an amount of about 9.5-10 percent by weight, sodium oxide and potassium oxide in combination in an amount of about 0-0.2 percent by weight, and iron oxide in an amount of about 0-0.1 percent by weight.
